(19) Europäisches Patentamt European Patent Office Office européen des brevets

(11) **EP 2 663 185 B1**

(12) **EUROPEAN PATENT SPECIFICATION**

(45) Date of publication and mention
of the grant of the patent:
**01.02.2017 Bulletin 2017/05**

(51) Int Cl.:
*A01N 37/00* (2006.01)       *A01N 37/10* (2006.01)
*A61K 31/215* (2006.01)      *A61K 31/235* (2006.01)
*A61P 3/04* (2006.01)        *A61P 29/00* (2006.01)
*A61P 43/00* (2006.01)

(21) Application number: **12865987.7**

(22) Date of filing: **10.01.2012**

(86) International application number:
**PCT/US2012/020718**

(87) International publication number:
**WO 2013/109241 (25.07.2013 Gazette 2013/30)**

(54) **CALEBIN A FOR USE IN THE TREATMENT OF INFLAMMATION AND OBESITY**

CALEBIN A ZUR BEHANDLUNG DER ENTZÜNDUNG UND DER FETTSUCHT

CALÉBINE A DESTINÉE AU TRAITEMENT DE L'INFLAMMATION ET DE L'OBÉSITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.01.2011 US 201161431147 P**

(43) Date of publication of application:
**20.11.2013 Bulletin 2013/47**

(73) Proprietor: **Majeed, Muhammed
East Windsor, NJ 08520-5321 (US)**

(72) Inventor: **PANDEY, Anjali
Bangalore 560058 (IN)**

(74) Representative: **Dr. Gassner & Partner mbB
Marie-Curie-Str. 1
91052 Erlangen (DE)**

(56) References cited:
**US-B1- 6 887 898     US-B2- 7 572 829
US-B2- 7 728 043**

• LI Y ET AL: "Calebin-A induces apoptosis and
modulates MAPK family activity in drug resistant
human gastric cancer cells", EUROPEAN
JOURNAL OF PHARMACOLOGY, ELSEVIER
SCIENCE, NL, vol. 591, no. 1-3, 4 September 2008
(2008-09-04), pages 252-258, XP023782821, ISSN:
0014-2999, DOI: 10.1016/J.EJPHAR.2008.06.065
[retrieved on 2008-06-22]
• LINI ALAPPAT ET AL: "Curcumin and obesity:
evidence and mechanisms", NUTRITION
REVIEWS, vol. 68, no. 12, 23 November 2010
(2010-11-23), pages 729-738, XP055103485, ISSN:
0029-6643, DOI:
10.1111/j.1753-4887.2010.00341.x

## Description

The invention in general relates to medicaments for obesity management. More specifically, it relates to anti-obesity potential of Calebin A.

## DESCRIPTION OF PRIOR ART

Obesity is the most prevalent nutritional disorder in industrialized countries and is a growing problem in developing countries. It is described as a global epidemic and overweight and obese individuals (BMI of 25 and above) are at increased risk for various chronic physical ailments and psychological problems such as depression, eating disorders and low self esteem. It is associated with various diseases like cardiovascular diseases, diabetes mellitus, osteoarthritis, obstructive sleep apnea and cancer. WHO considers obesity to be one of the top 10 causes of preventable death worldwide.

In obesity, there is an increase in the adipose tissue mass due to the production of new fat cells (adipocytes) through the process of adipogenesis and/or the deposition of increased amounts of cytoplasmic triglyceride per cell. A fat cell develops as internally produced lipid droplets coalesce into a single large mass. Eventually, cellulite results due to enhanced adipogenesis and accumulation of chunks of adipocytes under the skin dermis.

Studies of adipogenesis have proceeded with the hope that manipulation of this process in humans might lead to a reduction in the burden of obesity and diabetes. At molecular level, several markers have been targeted in treating obesity such as leptin, adiponectin, TNF-$\alpha$ etc..

Though drugs are available for treating the disorder, there is a constant need and search for safe natural approach to help manage obesity and its related socio-economic consequences.

Alappat, L. and Awad, A. B., Nutrition Reviews, Vol. 68(12), pages 729 to 738 addresses the evidence and mechanisms by which curcumin may play a role in downregulating obesity and reducing the impact of associated problems. The publication also addresses the effect of curcumin in inflammation. It does not disclose any effect of Calebin A.

Calebin A is known to protect neuronal cells from $\beta$-amyloid insult (Park SY et al, J Nat Prod. 2002 Sep; 65(9):1227-31), induce apoptosis and modulate MAPK family activity in drug resistant human gastric cancer cells (Li, Y. et al., Eur. J. Pharmacol. 2008 Sep 4; 591(1-3):252-8). In Li, Y. et al., Eur. J. Pharmacol. 2008 Sep 4; 591(1-3):252-8 it is also proposed to use Calebin A in the treatment of human gastric and other multidrug resistant cancers. A use in inhibiting inflammation caused by pro-inflammatory cytokines is not disclosed and obesity is not mentioned in the document. Zeng Y et al.(Chem Pharm Bull (Tokyo) 2007 Jun; 55(6):940-3) discusses two new calebin derivatives, 4"-(4'''-hydroxyphenyl-3'''-methoxy)-2"-oxo-3"-butenyl-3-(4'-hydroxyphenyl)-propenoate and 4"-(4'''-hydroxyphenyl)-2"-oxo-3"-butenyl-3-(4'-hydroxyphenyl-3'-methoxy)-propenoate.

The present invention discloses the potential of Calebin A to prevent fat accumulation during the terminal differentiation of adipocytes (fat cells) and applications thereof in obesity management. The present invention elucidates the potential of Calebin A to favorably modulate biochemical markers associated with obesity. Notable biomodulatory properties of Calebin A include inhibiting leptin production, increasing adiponectin expression and inhibiting local (adipocyte) and systemic inflammation caused by pro-inflammatory cytokines Tumor Necrosis Factor (TNF-$\alpha$), Interleukin-6 (IL-6) and Interleukin-1 (IL-1$\beta$).

Accordingly, it is the principle objective of the present invention to disclose anti-obesity potential of Calebin A.

The invention fulfills the aforesaid principle objective and provides further related advantages.

## SUMMARY OF THE INVENTION

The present invention discloses the potential of Calebin A in inhibiting adipogenesis and applications thereof in obesity management. The present invention elucidates the potential of Calebin A to favorably modulate biochemical markers associated with obesity in mammals. Notable biomodulatory properties of Calebin A include inhibiting leptin production, increasing adiponectin expression and inhibiting local (adipocyte) and systemic inflammation caused by pro-inflammatory cytokines Tumor Necrosis Factor (TNF-$\alpha$), Interleukin-6 (IL-6) and Interleukin-1 (IL-1$\beta$).

Other features and advantages of the present invention will become apparent from the following more detailed description, taken in conjunction with the accompanying drawings, which illustrate, by way of example, the principle of the invention.

## BRIEF DESCRIPTION OF FIGURES

**Fig.1** shows the graphical representation of the percentage adipogenesis inhibition effected by Calebin A at concentrations of 0.5, 1.0 and 2.0 $\mu$g/ml as studied by the Oil-Red-O-Staining method.

**Fig.2** shows the graphical representation of the percentage inhibition of leptin production in human adipocytes effected by Calebin A at concentrations of 0.5, 1.0 and 2.0 $\mu$g/ml. P value * : < 0.01; ** : <0.001.

**Fig.3** shows the graphical representation of the percentage increase of adiponectin expression in human adipocytes effected by Calebin A at concentrations of 0.5, 1.0 and 2.0 $\mu$g/ml. P value * : < 0.01.

**Figs.4 and 5** shows the graphical representation of the percentage inhibition of TNF-$\alpha$ expression (P value *: < 0.01; ** : <0.001) and IL-6 expression (P value * : < 0.01) respectively, in human adipocytes effected by Calebin A at concentrations of 0.5, 1.0 and 2.0 $\mu$g/ml.

**Fig.6** shows the graphical representation of the effect of multiple dose of Calebin A on the expression of TNF-$\alpha$ and IL-1$\beta$ in the serum from treated Swiss Albino mice. No. of animals = 6 per group, P-value: * < 0.01; ** < 0.001 students't' test.

**Fig.7** shows the graphical representation of the effect of multiple dose of Calebin A on the expression of IL-6 in the serum from treated Swiss Albino mice. No. of animals = 6 per group, P-value: * < 0.01; ** < 0.001 students't' test.

## DETAILED DESCRIPTION OF INVENTION

The present invention discloses the potential of Calebin A to prevent fat accumulation during the terminal differentiation of adipocytes (fat cells) and applications thereof in obesity management. The present invention elucidates the potential of Calebin A to favorably modulate biochemical markers associated with obesity. Notable biomodulatory properties of Calebin A include inhibiting leptin production, increasing adiponectin expression and inhibiting local (adipocyte) and systemic inflammation caused by pro-inflammatory cytokines Tumor Necrosis Factor (TNF-$\alpha$), Interleukin-6 (IL-6) and Interleukin-1 (IL-1$\beta$).

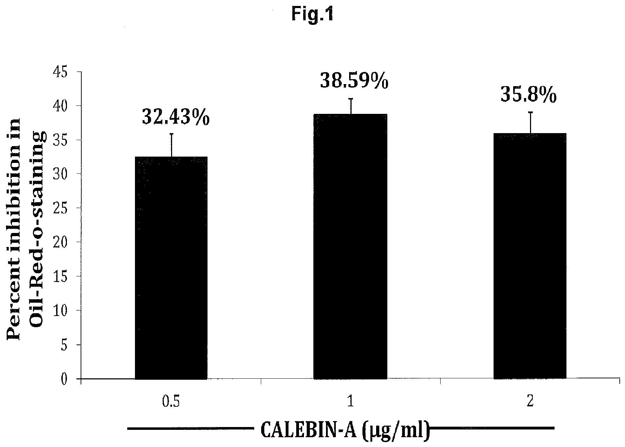

In the most preferred embodiment, the present invention relates to a method of inhibiting adipogenesis in vitro, said method comprising step of bringing into contact the adipocytes with an effective amount of Calebin A. In other words, the present invention relates to a method of preventing accumulation of fat during the terminal differentiation of mammalian adipocytes. (**Fig.1**).

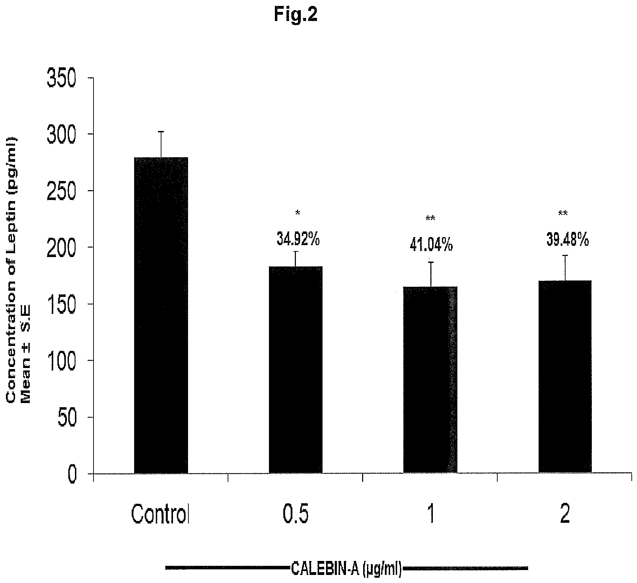

In another preferred embodiment, the present invention relates to a method of inhibiting leptin expression in adipocytes in vitro, said method comprising step of bringing into contact the adipocytes with an effective amount of Calebin A (**Fig.2**).

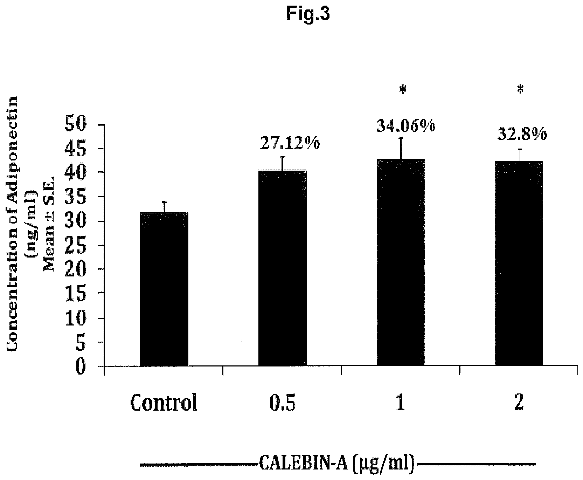

In another preferred embodiment, the present invention relates to a method of increasing the expression of adiponectin in adipocytes in vitro, said method comprising step of bringing into contact the adipocytes with an effective amount of Calebin A (**Fig.3**).

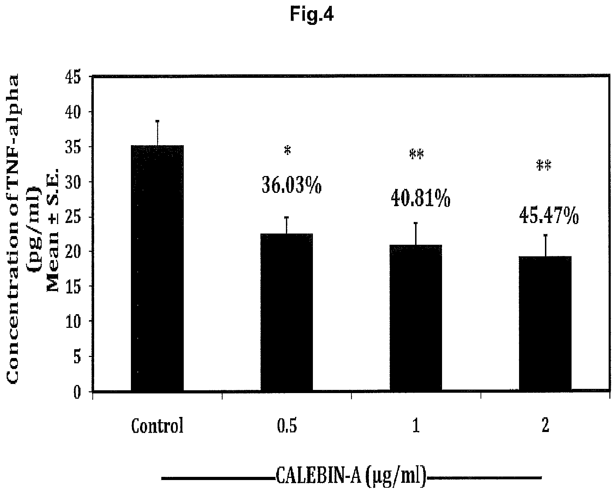

In another preferred embodiment, the present invention relates to a method of inhibiting pro-inflammatory cytokine TNF- $\alpha$ expression in adipocytes in vitro, said method comprising step of bringing into contact the adipocytes with an effective amount of Calebin A (**Fig.4**).

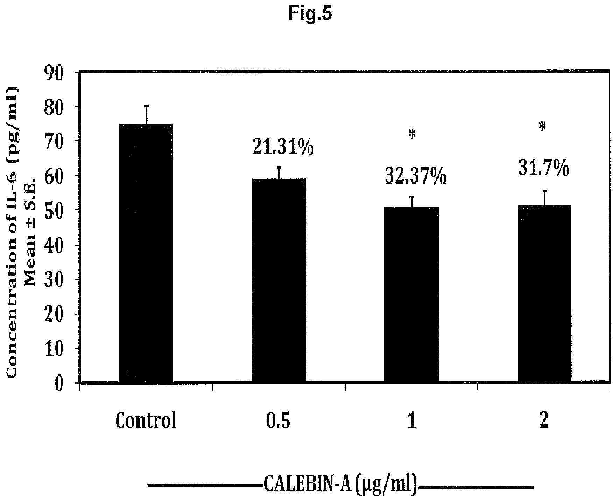

In yet another preferred embodiment, the present invention relates to a method of inhibiting pro-inflammatory cytokine Interleukin-6 expression in adipocytes in vitro, said method comprising step of bringing into contact the adipocytes with an effective amount of Calebin A (**Fig.5**).

In specific embodiment, the adipocytes referred to herein above are human adipocytes.

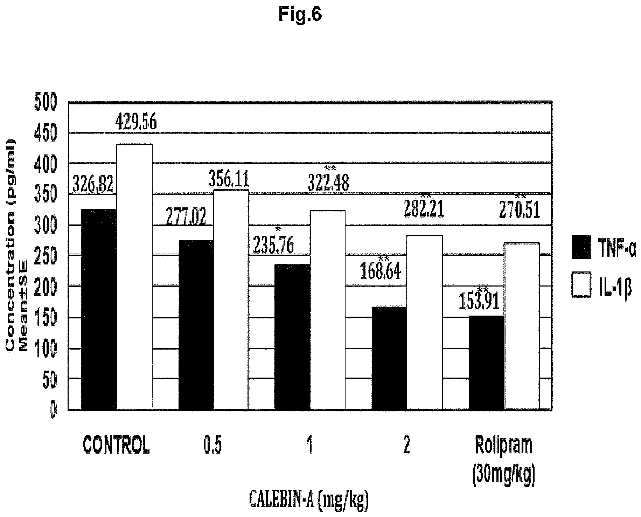

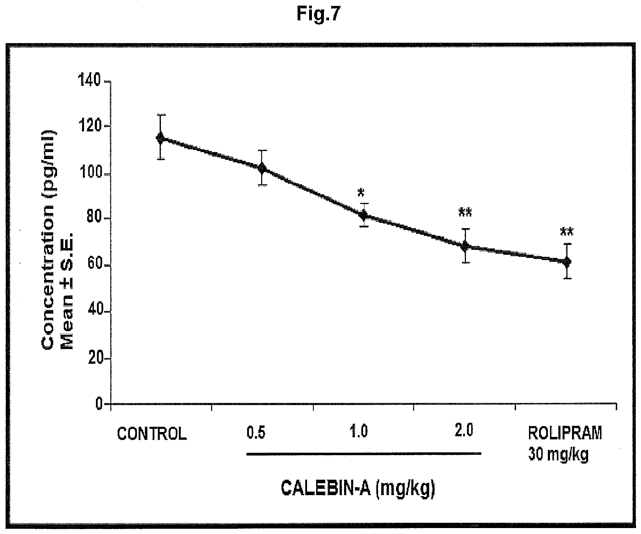

In yet another preferred embodiment, the present invention relates to Calebin A for use in inhibiting local and systemic inflammation caused by pro-inflammatory cytokines Tumor Necrosis Factor-$\alpha$ (TNF-$\alpha$), Interleukin-6 (IL-6) and Interleukin-1$\beta$ (IL-1$\beta$) **[Figs 6 and 7]**.

In yet another preferred embodiment, the present invention relates to Calebin A for use in obesity management.

In yet another preferred embodiment, the obesity management is mammalian obesity management.

In yet another preferred embodiment, the obesity management is human obesity management.

The potential therapeutic value of Calebin A as an anti-obesity molecule may be understood through specific examples elucidated herein below.

## EXAMPLE I

**Acute Oral Toxicity of Calebin A**

**Table I** lists the parameters studied for Acute Oral Toxicity of Calebin A.

**Results:** No mortality was observed up to 2000mg/kg p.o. in mice up to two weeks of observation.

**Table I:** parameters studied for Acute Oral Toxicity of Calebin A

| General Behavior | Dermal |
|---|---|
| Aggression=Nil | Blanching=Nil |
| Fear=Nil | Hyperaemia=Nil |
| Passive=Nil | Cyanosis=Nil |
| General Movement=Normal | |
| General Locomotor Activity=Normal | |
| **Central Nervous System** | **General Observations** |
| Excitation=Nil | Muscular Weakness=Nil |
| Motor Activity=Nil | Salivation=Nil |
| Tremors=Nil | Pilo-erection=Nil |
| Clonic Convulsions=Nil | Diarrhea=Nil |
| Tonic Convulsions=Nil | |
| **Respiratory System** | **Reflexes** |
| Respiration Rate=Normal | Corneal=No effect |
| Respiration Depth=Normal | Pinnal=No effect |
| **Autonomic Nervous System** | **Food and Water (Intake and Excretion)** |
| Motor activity=Normal | Fecal Output=Normal |
| Atexia=Nil | Urine Output=Normal |
| Respiration Rate=Normal | |
| Diarrhea=Nil | |

## EXAMPLE II

**Oil-Red-O-Staining of adipogenic cultures and estimation of leptin, adiponectin, TNF-$\alpha$ and IL-6 by ELISA.**

Terminal differentiation of adipocytes is accompanied by the accumulation of great amounts of lipids in large cytoplasmic vesicles. A common assay to measure adipocyte differentiation in cell culture is with the dye Oil Red-O (ORO). ORO is a lipid-soluble bright red dye which is a reliable indicator of adipocyte differentiation (adipogenesis).

**Principle:**

Oil Red O (Solvent Red 27, Sudan Red 5B, C.I. 26125, and $C_{26}H_{24}N_4O$) is a lysochrome (fat-soluble dye) diazo dye used for staining of neutral triglycerides and lipids on frozen sections and some lipoproteins on paraffin sections. It has the appearance of a red powder with maximum absorption at 518(359) nm. Oil Red O is one of the dyes used for Sudan staining. Similar dyes include Sudan III, Sudan IV, and Sudan Black B. The staining has to be performed on fresh samples, as alcohol fixation removes the lipids. Oil Red O largely replaced Sudan III and Sudan IV, as it provides much deeper red color and the stains are therefore much easier to see.

Oil red O is an oil soluble dye. Oil soluble dyes exhibit greater solubility of the dye in lipid substances in the tissues/cells, than in the usual hydro alcoholic dye solvents. Hence, it will deeply stain the cells.

**Methodology:**

3T3-L1 cells approximately 60 X 104 cells are seeded for 48-72 hrs to get 70-80% confluence. After 48 hrs 200 $\mu$l of AIM (Adipogenesis induction medium) freshly prepared is added. 72 hrs later 200 $\mu$l APM (Adipogenesis progression medium) with the test compounds in different concentrations is added to the wells. The cells are incubated for 48 hrs in a humidified atmosphere (370 C) of 5% CO2 and 95% air. The supernatant is collected and stored for the estimation of leptin, adiponectin, IL-6 and TNF-$\alpha$ by ELISA. Cells are fixed by adding 100 $\mu$l of 10% formalin and ORO staining is done. OD is read at 492 nm in microplate reader. The results are expressed as $IC_{50}$ values using Graphpad prism software.

The percentage of inhibition of adipogenesis is calculated as follows,

$$\% \text{ Inhibition} = \text{C-T}$$

$$\ldots\ldots\ldots \text{ X } 100$$

$$\text{T}$$

Where C-absorbance of Oil red O in differentiating/undifferentiated cells

T-absorbance of Oil red O in sample treated differentiating/undifferentiated cells. The estimation of leptin, adiponectin, IL-6 and TNF-$\alpha$ is done according to user's manual from R&D Systems.

**References:**

1. Wu Z, Xie Y, Morrison RF, Bucher NLR, Farmer SR 1998. PPAR $\gamma$ induces the Insulin-dependent Glucose Transporter GLUT4 in the absence of C/EBP□ during the conversion of 3T3 fibroblasts into adipocytes. J Clin Invest. 101:22-32.
2. A pre-adipose 3T3 cell variant highly sensitive to adipogenic factors & to human growth hormone. LA Salazar-Olivo, F Castro-Munozledo & W Kuri-Harcuch. Department of Cell Biology, Centro de Investigation y de Estudios Avanzados del I.P.N., Mexico D.F., Mexico. Journal of Cell Science, Vol 108, Issue 5 2101-2107.
3. A Nuclear Receptor Atlas: 3T3-L1 Adipogenesis. Mingui Fu, Tingwan Sun, Angie L. Bookout, Micheal Downes, Ruth T. Yu, Ronald M. Evans and David J. Mangelsdorf. Molecular Endocrinology 19 (10): 2437-2450.
4. Aimee D, Kohn etal, JBC, Vol 271, No. 49, pp-31372-31378.

**Result:**

**Fig.1** shows percentage adipogenesis inhibition of 32.43%, 38.59% and 35.8% respectively effected by Calebin A at concentrations of 0.5, 1.0 and 2.0 $\mu$g/ml studied by the Oil-Red-O-Staining method.

**Fig.2** shows percentage inhibition of leptin production (34.92%, 41.04% and 39.48% respectively) in human adipocytes by Calebin A at concentrations of 0.5, 1.0 and 2.0 $\mu$g/ml. The importance of the effects of Calebin A in inhibiting leptin production in human adipocytes and correlation thereof to obesity management stems from the following facts (Notes on Pathophysiology of the Endocrine System, Colorado State University).

Leptin is a protein hormone expressed predominantly in adipocytes. It has important effects in regulating body weight, metabolism and reproductive function. Encoded by the obese (ob) gene, the protein is approximately ~16 kDa in mass. At normal concentrations, Leptin's biological function is predominantly vested in its effects on hypothalamic centers of the brain that control hunger, appetite, regulation of body temperature and energy metabolism. Thus leptin, in a non-obese individual could result in weight loss by two important mechanisms. (i) Decrease in hunger and food consumption most probably through the inhibition of neuropeptide Y that controls feeding behavior and (ii) increase in energy expenditure through increased body temperature, oxygen consumption and loss of adipose tissue mass. However, excessive secretion of leptin as in case of obesity or experimental models of induced obesity leads to disrupted functions of hypothalamic centers that an obese subject fails to attain satiations and tends to go on a over feeding mode. Hence it becomes imperative to bring about effective reduction of the over excessive levels of leptin in obesity and Calebin A shows promise in this area as indicated in **Fig.2**.

**Fig.3** shows percentage enhancement of adiponectin expression (27.12%, 34.06% and 32.8% respectively) in human adipocytes by Calebin A at concentrations of 0.5, 1.0 and 2.0 $\mu$g/ml. Adiponectin is a cytokine produced almost exclusively by adipocytes and is expressed in very high levels by lean and healthy individuals. Obese individuals on the other hand express reduced levels of this adipokine and are prone to coronary heart disease (CAD), diabetes mellitus and hypertension.

**References**

1. Tamar.R.Aprahamian and Flora Sam, "Adiponectin in Cardiovascular Inflammation and Obesity, Int J Inflam. 2011; 2011: 376909;
2. Hotta K, Funahashi T, Arita Y, et al. Plasma concentrations of a novel, adipose-specific protein, adiponectin, in type 2 diabetic patients. Arteriosclerosis, Thrombosis and Vascular Biology. 2000; 20(6): 1595-1599;
3. Iwashima Y, Katsuya T, Ishikawa K, et al. Hypoadiponectinemia is an independent risk factor for hypertension. Hypertension. 2004; 43(6):1318-1323;
4. Kumada M, Kihara S, Sumitsuji S, et al. Association of hypoadiponectinemia with coronary artery disease in men. Arteriosclerosis, Thrombosis and Vascular Biology. 2003; 23(1):85-89 and
5. Lindsay RS, Funahashi T, Hanson RL, et al. Adiponectin and development of type 2 diabetes in the Pima Indian population. The Lancet. 2002; 360(9326):57-58.

Calebin A is shown (**Fig.3**) to effectively increase levels of adiponectin in human adipocytes and thus show promise in the area of obesity management.

**Figs. 4 and 5** show the percentage inhibition of TNF-$\alpha$ (36.03%, 40.81% and 45.47% respectively) and IL-6 (21.31%, 32.37% and 31.7% respectively) by Calebin A at concentrations of 0.5, 1.0 and 2.0 $\mu$g/ml. Bastard JP et al, "Recent Advances in the relationship between obesity, inflammation and insulin resistance", Eur Cytokine Netw. 2006 Mar;17(1):4-12 cite that obesity is associated with low-grade inflammation of the white adipose tissue (WAT). The authors also remark that in obesity, WAT is characterized by increased expression of pro-inflammatory molecules like TNF-$\alpha$ and IL-6 which not only exert effects on WAT but also on other systemic organs of the body. **Figs.4 and 5** demonstrate that Calebin A is effective in reducing TNF-$\alpha$ and IL-6 expression in adipocytes and would be a useful agent to modulate effects of local and systemic inflammation in obesity.

## EXAMPLE III

### Modulation of systemic inflammation by Calebin A

The present inventors also adduce extra evidence to support the ability of Calebin A to suppress intracellular TNF and extracellular IL-1$\beta$ in murine neutrophil systems (**Table II, Table III**). Neutrophils are isolated by histopaque gradient method tested for their ability to produce in vitro TNF-$\alpha$ following stimulation with Lipopolysaccharide (LPS). The cells were incubated with phycoerythrin (PE)-labeled antimouse TNF-$\alpha$. in the dark, and after being washed with sterile PBS, samples were resuspended in PBS (pH 7.4) and acquired directly on the flow cytometer (BDLSR; Becton Dickinson). A fluorescence trigger was set on the PE (FL1) parameter of the gated neutrophil populations (10,000 events). Rolipram at 100 $\mu$g/ml was used as standard inhibitor of TNF-$\alpha$ in this study. Fluorescence compensation, data analysis, and data presentation were performed using Cell Quest Pro software (Becton Dickinson).

**References**

1. Clara, B., R. C. Arancha, G. M. Andre's, P. Atanasio, A. Julia, and O. Alberto. 2003. A new method for detecting TNF-$\alpha$-secreting cells using direct immunofluorescence surface membrane stainings. J. Immuno. Methods 264:77-87.
2. Khurshid A. Bhat, Bhahwal A. Shah, Kuldeep K. Gupta, Anjali Pandey, Sarang Bani, Subhash C. Taneja. Semi-synthetic analogs of pinitol as potential inhibitors of TNF-$\alpha$ cytokine expression in human neutrophils. Bioorganic & Medicinal Chemistry Letters 19 2009, 1939-1943.

**TABLE II**

| Serial No | Sample | Concentration (µg/ml) | Expression of TNF-$\alpha$ Mean $\pm$ S.E | % Activity |
|---|---|---|---|---|
| 1 | LPS Control | - | 2.62$\pm$0.01 | - |
| 2 | Calebin A | 0.5 | 1.87$\pm$0.04* | 28.62%$\downarrow$ |
| 3 | Calebin A | 1.0 | 1.70$\pm$0.02** | 35.11%$\downarrow$ |
| 4 | Calebin A | 2.0 | 1.59$\pm$0.05** | 39.31%$\downarrow$ |
| 5 | Rolipram | 100 | 0.73$\pm$0.09** | 72.13%$\downarrow$ |
| %$\downarrow$: indicates suppression of TNF-$\alpha$ expression<br>No. of observations = 3<br>P-value: * < 0.01; ** < 0.001 students 't' test | | | | |

**TABLE III**

| Samples Treatment | Concentration (pg/ml) | % Activity |
|---|---|---|
| LPS Control | 51.80 $\pm$ 2.18 | - |
| Calebin-A<br>0.5µg/ml<br>1.0 µg/ml<br>2.0 µg/ml | 41.24 $\pm$ 1.16*<br>39.26 $\pm$ 2.52*<br>37.16 $\pm$ 2.11** | 20.38%$\downarrow$<br>24.20%$\downarrow$<br>28.26%$\downarrow$ |
| Rolipram (Standard)<br>100 µg/ml | 22.52$\pm$ 1.60** | 56.52%$\downarrow$ |
| %$\downarrow$: indicates suppression of IL-1 $\beta$ expression<br>No. of observations = 3<br>P-value: * < 0.01; ** < 0.001 students 't' test | | |

The present inventors also adduce study data on the ability of Calebin-A to reduce expression of Extracellular TNF-$\alpha$, IL-1 beta [**Fig.6**] and IL-6 [**Fig.7**] in serum from treated mice (in-vivo models). Swiss albino male mice aged 6-8 weeks were maintained at 22$\pm$ 2 0C under 12/12 h light dark cycle. Mice received oral treatment of test drugs at graded doses (w/v) for 6 days, followed by intravenous injection of 1 mg/kg of LPS according to the method described by Brieva A, Guerrero A, Alonso-Lebrero J L and Pivel JP. 2001. Inmunoferon, a glycoconjugate of natural origin, inhibits LPS-induced TNF-$\alpha$ production and inflammatory responses. International Immunopharmacology 1, 1979-1987. Six mice were employed in each group and experiments were performed in triplicates. TNF-$\alpha$, IL-1 beta and IL-6 production was evaluated by a commercial ELISA kits (R&D Systems) in serum from treated mice, 90 min after LPS injection. Rolipram at 30 mg/kg was used as standard drug.

**Figs.6 and 7** demonstrate that Calebin A is effective in reducing TNF-$\alpha$, IL-1 beta and IL-6 thus indicating that the compound is a useful agent to modulate effects of local and systemic inflammation in obesity.

**Claims**

1. Calebin A for use in inhibiting local and systemic inflammation caused by pro-inflammatory cytokines Tumor Necrosis Factor-$\alpha$ (TNF-$\alpha$), Interleukin-6 (IL-6) and Interleukin-1$\beta$ (IL-1$\beta$).

2. Calebin A for use in obesity management.

3. The Calebin A for use as claimed in claim 2, wherein the obesity management is mammalian obesity management.

4. The Calebin A for use as claimed in claim 2, wherein the obesity management is human obesity management.

5. A method of inhibiting adipogenesis in vitro, said method comprising step of bringing into contact the adipocytes with an effective amount of Calebin A.

6. A method of inhibiting leptin expression in adipocytes in vitro, said method comprising step of bringing into contact the adipocytes with an effective amount of Calebin A.

7. A method of increasing the expression of adiponectin in adipocytes in vitro, said method comprising step of bringing into contact the adipocytes with an effective amount of Calebin A.

8. A method of inhibiting obesity induced pro-inflammatory cytokine expression in adipocytes in vitro, said method comprising step of bringing into contact the adipocytes with an effective amount of Calebin A, wherein the pro-inflammatory cytokine is Tumor Necrosis Factor-$\alpha$ (TNF-$\alpha$) or Interleukin-6 (IL-6).

9. The method according to claim 5, 6, 7, or 8, wherein the adipocytes are human adipocytes.


**Patentansprüche**

1. Calebin A zur Verwendung beim Hemmen lokaler und systemischer Entzündung, verursacht durch die pro-inflammatorischen Cytokine Tumornekrosefaktor-$\alpha$ (TNF-$\alpha$), Interleukin-6 (IL-6) und Interleukin-1$\beta$ (IL-1$\beta$).

2. Calebin A zur Verwendung beim Fettsucht-Management.

3. Calebin A zur Verwendung nach Anspruch 2, wobei das Fettsucht-Management ein Management einer Säugetier-Fettsucht ist.

4. Calebin A zur Verwendung nach Anspruch 2, wobei das Fettsucht-Management ein Management einer menschlichen Fettsucht ist.

5. Verfahren zur Hemmung von Fettbildung in vitro, wobei das Verfahren den Schritt des Inkontaktbringens der Fettzellen mit einer wirksamen Menge von Calebin A umfasst.

6. Verfahren zur Hemmung der Expression von Leptin in Fettzellen in vitro, wobei das Verfahren den Schritt des Inkontaktbringens der Fettzellen mit einer wirksamen Menge von Calebin A umfasst.

7. Verfahren zur Erhöhung der Expression von Adiponektin in Fettzellen in vitro, wobei das Verfahren den Schritt des Inkontaktbringens der Fettzellen mit einer wirksamen Menge von Calebin A umfasst.

8. Verfahren zur Hemmung von durch Fettsucht ausgelöste Expression von pro-inflammatorischem Cytokin in Fettzellen in vitro, wobei das Verfahren den Schritt des Inkontaktbringens der Fettzellen mit einer wirksamen Menge von Calebin A umfasst, wobei das pro-inflammatorische Cytokin Tumornekrosefaktor-$\alpha$ (TNF-$\alpha$) oder Interleukin-6 (IL-6) ist.

9. Verfahren nach Anspruch 5, 6, 7 oder 8, wobei die Fettzellen menschliche Fettzellen sind.


**Revendications**

1. Calébine A destinée à être utilisée dans l'inhibition de l'inflammation locale et systémique provoquée par des cytokines pro-inflammatoires facteur de nécrose tumorale-$\alpha$ (TNF-$\alpha$), interleukine-6 (IL-6) et interleukine-1$\beta$ (IL-1$\beta$).

2. Calébine A destinée à être utilisée dans la gestion de l'obésité.

3. Calébine A destinée à être utilisée selon la revendication 2, dans laquelle la gestion de l'obésité est la gestion de l'obésité mammifère.

4. Calébine A destinée à être utilisée selon la revendication 2, dans laquelle la gestion de l'obésité est la gestion de l'obésité humaine.

5. Procédé d'inhibition de l'adipogenèse in vitro, ledit procédé comprenant l'étape consistant à amener en contact les adipocytes avec une quantité efficace de calébine A.

6. Procédé d'inhibition de l'expression de leptine dans des adipocytes in vitro, ledit procédé comprenant l'étape consistant à amener en contact les adipocytes avec une quantité efficace de calébine A.

7. Procédé d'augmentation de l'expression d'adiponectine dans des adipocytes in vitro, ledit procédé comprenant l'étape consistant à amener en contact les adipocytes avec une quantité efficace de calébine A.

8. Procédé d'inhibition de l'expression de cytokine pro-inflammatoire induite par l'obésité dans des adipocytes in vitro, ledit procédé comprenant l'étape consistant à amener en contact les adipocytes avec une quantité efficace de calébine A, dans lequel la cytokine pro-inflammatoire est le facteur de nécrose tumorale-$\alpha$ (TNF-$\alpha$) ou l'interleukine-6 (IL-6).

9. Procédé selon la revendication 5, 6, 7 ou 8, dans lequel les adipocytes sont des adipocytes humains.

## Fig.1

**Fig.2**

Fig.3

Fig.4

Fig.5

**Fig.6**

CALEBIN-A (mg/kg)

**Fig.7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ALAPPAT, L. ; AWAD, A. B.** *Nutrition Reviews,* vol. 68 (12), 729-738
- **PARK SY et al.** *J Nat Prod.,* September 2002, vol. 65 (9), 1227-31
- **LI, Y. et al.** *Eur. J. Pharmacol.,* 04 September 2008, vol. 591 (1-3), 252-8
- **ZENG Y et al.** *Chem Pharm Bull (Tokyo),* June 2007, vol. 55 (6), 940-3
- **WU Z ; XIE Y ; MORRISON RF ; BUCHER NLR ; FARMER SR.** PPAR$\gamma$ induces the Insulin-dependent Glucose Transporter GLUT4 in the absence of C/EBP□ during the conversion of 3T3 fibroblasts into adipocytes. *J Clin Invest.,* 1998, vol. 101, 22-32
- A pre-adipose 3T3 cell variant highly sensitive to adipogenic factors & to human growth hormone. **LA SALAZAR-OLIVO ; F CASTRO-MUNOZLEDO ; W KURI-HARCUCH.** Journal of Cell Science. Department of Cell Biology, Centro de Investigation y de Estudios Avanzados del I.P.N, vol. 108, 2101-2107
- A Nuclear Receptor Atlas: 3T3-L1 Adipogenesis. **MINGUI FU ; TINGWAN SUN ; ANGIE L. BOOKOUT ; MICHEAL DOWNES ; RUTH T. YU ; RONALD M. EVANS ; DAVID J. MANGELSDORF.** Molecular Endocrinology. vol. 19, 2437-2450
- **AIMEE D ; KOHN ETAL et al.** *JBC,* vol. 271 (49), 31372-31378
- **TAMAR.R.APRAHAMIAN ; FLORA SAM.** Adiponectin in Cardiovascular Inflammation and Obesity. *Int J Inflam.,* 2011, vol. 2011, 376909
- **HOTTA K ; FUNAHASHI T ; ARITA Y et al.** Plasma concentrations of a novel, adipose-specific protein, adiponectin, in type 2 diabetic patients. *Arteriosclerosis, Thrombosis and Vascular Biology,* 2000, vol. 20 (6), 1595-1599

- **IWASHIMA Y ; KATSUYA T ; ISHIKAWA K et al.** Hypoadiponectinemia is an independent risk factor for hypertension. *Hypertension,* 2004, vol. 43 (6), 1318-1323
- **KUMADA M ; KIHARA S ; SUMITSUJI S et al.** Association of hypoadiponectinemia with coronary artery disease in men. *Arteriosclerosis, Thrombosis and Vascular Biology,* 2003, vol. 23 (1), 85-89
- **LINDSAY RS ; FUNAHASHI T ; HANSON RL et al.** Adiponectin and development of type 2 diabetes in the Pima Indian population. *The Lancet,* 2002, vol. 360 (9326), 57-58
- **BASTARD JP et al.** Recent Advances in the relationship between obesity, inflammation and insulin resistance. *Eur Cytokine Netw.,* March 2006, vol. 17 (1), 4-12
- **CLARA, B. ; R. C. ARANCHA ; G. M. ANDRE'S ; P. ATANASIO ; A. JULIA ; O. ALBERTO.** A new method for detecting TNF-$\alpha$-secreting cells using direct immunofluorescence surface membrane stainings. *J. Immuno. Methods,* 2003, vol. 264, 77-87
- **KHURSHID A ; BHAT, BHAHWAL A ; SHAH, KULDEEP K ; GUPTA, ANJALI PANDEY ; SARANG BANI ; SUBHASH C. TANEJA.** Semi-synthetic analogs of pinitol as potential inhibitors of TNF-$\alpha$ cytokine expression in human neutrophils. *Bioorganic & Medicinal Chemistry Letters,* 2009, vol. 19, 1939-1943
- **BRIEVA A ; GUERRERO A ; ALONSO-LEBRERO J L ; PIVEL JP.** Inmunoferon, a glycoconjugate of natural origin, inhibits LPS-induced TNF-$\alpha$ production and inflammatory responses. *International Immunopharmacology,* 2001, vol. 1, 1979-1987